# EUROPEAN PATENT APPLICATION

(11) **EP 3 769 803 A2**
(43) Date of publication of application: **27.01.2021**
(21) Application number: 20182045.3
(22) Date of filing: 24.06.2020
(51) Int. Cl.: A61M 25/00

(54) **MULTI-LUMEN CATHETER**

(30) Priority: 24.06.2019 US 201962865482 P
(71) Applicant: ORBUSNEICH MEDICAL PTE. LTD, 079906 Singapore (SG)
(72) Inventor: COTTONE JR., Robert John, DAVIE, FL 33330 (US)
(74) Representative: Jilderda, Anne Ayolt

(57) **Abstract**

A medical device (10), including an elongate catheter body (12) defining a proximal segment (14), a distal segment (18), and a first lumen (20) therethrough; a tube (24) attached to the distal segment of the catheter body, the tube defining a proximal end, a distal end, and a second lumen (26) therethrough; wherein the proximal end of the tube is attached to the catheter body at a proximal joint (28); wherein the distal end of the tube is attached to the catheter body at a distal joint (30); and wherein a portion of the tube extending between the first and second joints is movable with respect to the catheter body.

## Description

### FIELD OF THE INVENTION

The present disclosure relates to intravascular medical devices and methods of use thereof.

### BACKGROUND OF THE INVENTION

Various catheter systems have been developed for the intravascular delivery of medical devices, such as stents or angioplasty balloons. Typically, a guide wire is introduced into a vessel and advanced through the vasculature to a treatment site. A catheter is then advanced over the guide wire so that the distal end of the catheter is located at the treatment site. The catheter and/or guidewire then may be used to transport and place any of a variety of medical devices, such as stents, grafts, angioplasty balloons, atherectomy devices, etc., in proximity to the treatment site.

The success of many minimally invasive medical procedures relies on the ability to precisely position such medical devices within such a targeted tissue region. Such precise positing can be complicated if the treatment requires accessing and navigating a bifurcation in the targeted vasculature. The present disclosure provides devices and methods of use thereof for improving access and navigation of such vascular bifurcations and other multiple tortuous pathways.

### SUMMARY OF THE INVENTION

The present invention advantageously provides a medical device, including an elongate catheter body defining a proximal segment, a distal segment, and a first lumen therethrough; a tube attached to the distal segment of the catheter body, the tube defining a proximal end, a distal end, and a second lumen therethrough; wherein the proximal end of the tube is attached to the catheter body at a first joint; wherein the distal end of the tube is attached to the catheter body at a second joint; and wherein a portion of the tube extending between the first and second joints is movable with respect to the catheter body.

The first joint may include a first cap concentrically mounted onto the proximal end of the tube, wherein the first cap is attached to the catheter body. The second joint may include a second cap concentrically mounted onto the distal end of the tube, and the second cap may be attached to the catheter body. At least one of the first and second joints may include attachment of the tube to the catheter body with heat shrink tubing. At least one of the first and second joints may include attachment of the tube to the catheter body with an adhesive. The tube may be at least partially constructed from a polymer, and at least one of the first and second joints may include attachment of the tube to the catheter body by melt fusing a portion of the tube to a portion of the catheter body. The first lumen may not be concentric with the second lumen.

The medical device may further comprise a deflection element attached to the distal segment of the catheter body, and the deflection element may define an arcuate surface positioned adjacent to the first lumen. The arcuate surface may define an arc between 45 degrees and 135 degrees.

The deflection element may define a first opening substantially coaxial with the first lumen, a second opening that is substantially perpendicular to the first opening, and the arcuate surface may extend between the first and second openings.

The deflection element may define a third opening substantially perpendicular to each of the first and second openings. The deflection element may be radiopaque, and/or may be movable with respect to the catheter body. The deflection element may be movable along a longitudinal axis of the catheter body.

The present disclosure also provides a medical device, having an elongate catheter body defining a proximal segment, a distal segment, and a first lumen therethrough; a tube attached to the distal segment of the catheter body, the tube defining a second lumen therethrough that is not concentric with the first lumen; and a deflection element attached to the distal segment of the catheter body, the deflection element defining an arcuate surface positioned adjacent to a distal end of the first lumen, wherein the arcuate surface defines an arc between 45 degrees and approximately 135 degrees.

The deflection element may define a first opening substantially coaxial with the first lumen, a second opening that is substantially perpendicular to the first opening, and the arcuate surface may extend between the first and second openings. The deflection element may define a third opening substantially perpendicular to each of the first and second openings. The deflection element may be radiopaque and/or may be movable with respect to the catheter body. The deflection element is movable along a longitudinal axis of the catheter body. At least a portion of the tube may be movable independently of the catheter body.

### BRIEF DESCRIPTION OF THE DRAWINGS

A more complete understanding of the present disclosure, and the attendant advantages and features thereof, will be more readily understood by reference to the following detailed description when considered in conjunction with the accompanying drawings wherein:
- FIG. 1: is an illustration of an example of a medical device constructed in accordance with the principles of the present disclosure;
- FIG. 2: is a closer view of the distal segment of the medical device of FIG. 1;
- FIG. 3: is a side view of the distal segment of the medical device of FIG. 1;
- FIG. 4: is another view of the distal segment of the medical device of FIG. 1;
- FIG. 5: is a distal end view of the medical device of FIG. 1;
- FIG. 6: is a proximal end view of the medical device of FIG. 1;
- FIG. 7: is a side view of a bowed configuration of an example of a medical device constructed in accordance with the principles of the present disclosure;
- FIG. 8: is a perspective view of the bowed configuration of the example of a medical device shown in FIG. 7;
- FIG. 9: is a front view of the bowed configuration of the example of a medical device shown in FIG. 7;
- FIG. 10: is an illustration of another example of a medical device constructed in accordance with the principles of the present disclosure;
- FIG. 11: is an illustration of another example of a medical device constructed in accordance with the principles of the present disclosure;
- FIG. 12: is a side view of the distal segment of the medical device of FIG. 11;
- FIG. 13: is a cross-sectional view of the distal segment of the medical device of FIG. 11;
- FIG. 14a-b: are illustrations of an example of a deflection element of the medical device of FIG. 11;
- FIG. 15: is an illustration of another example of a deflection element of a medical device constructed in accordance with the principles of the present disclosure;
- FIG. 16: is a proximal end view of the medical device of FIG. 11;
- FIG. 17: is a distal end view of the medical device of FIG. 11;
- FIG. 18: is another view of the distal segment of the medical device of FIG. 11;
- FIG. 19: is yet another view of the distal segment of the medical device of FIG. 11;
- FIG. 20: is an illustration of a medical device with the deflection element of FIG. 15 constructed in accordance with the principles of the present disclosure; and
- FIG. 21: is another view of the distal segment of the medical device of FIG. 20.

### DETAILED DESCRIPTION OF THE INVENTION

The present disclosure provides intravascular medical devices and methods of use thereof. In particular, the present disclosure provides multi-lumen medical devices operable to facilitate procedures using multiple guywires or other auxiliary devices that are manipulated independently and/or along different segments of the vasculature.

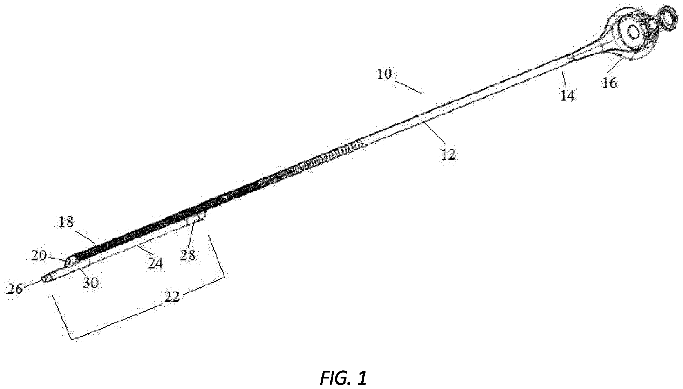

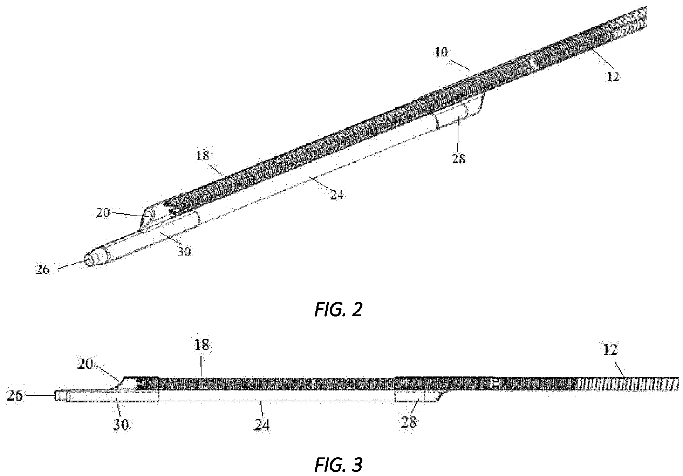

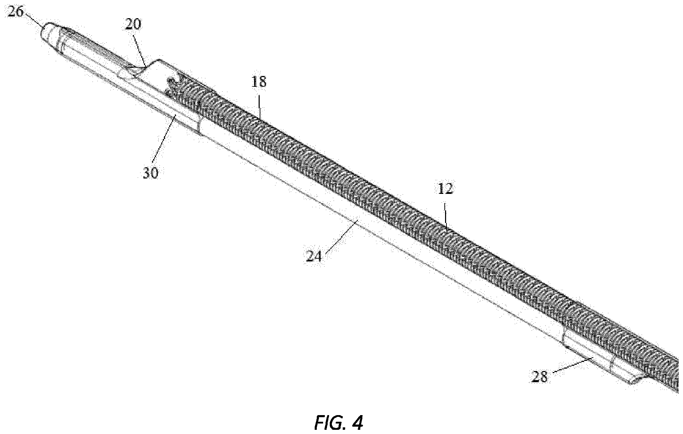

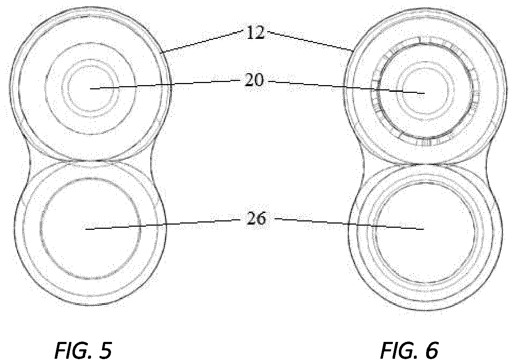

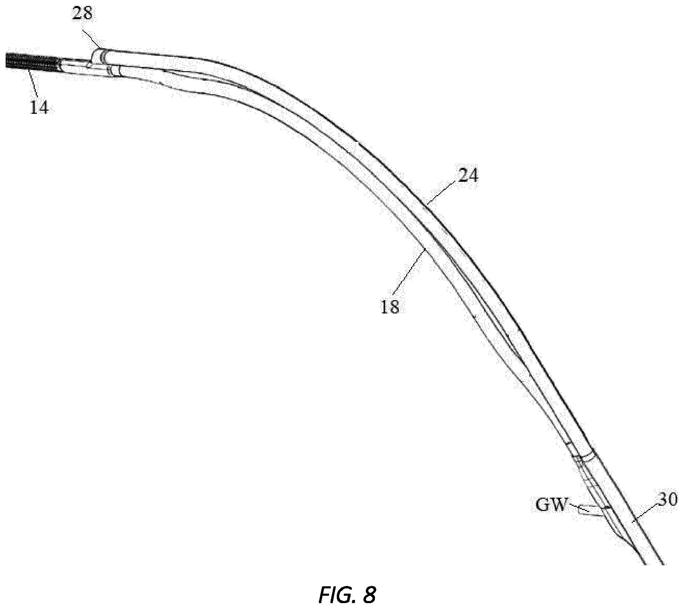

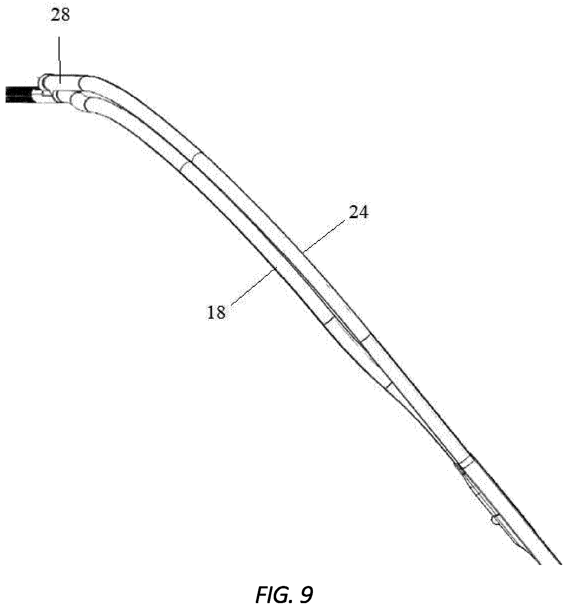

Now referring to the figures, FIGS. 1-9 illustrate an example of an intravascular medical device 10, such as a catheter, constructed in accordance with the principles and advantages disclosed herein. The device 10 is a minimally-invasive device that can be introduced and operated intravenously in conjunction with one or more other devices as disclosed herein, such as those used in interventional cardiology to assess and/or treat occlusions or other vascular defects or conditions. The device 10 generally includes an elongated catheter body 12 with sufficient length, flexibility, and torqueability characteristics to be introduced and operated from an exterior of the patient, traverse the vasculature, and be positioned proximate the region being assessed or treated. The catheter body 12 generally includes a proximal segment 14 that may connect to and/or terminate at a hub 16, and a distal segment 18. The catheter body 12 further includes or defines a first lumen 20 extending therethrough and exiting at the distal segment 18, where the lumen 20 has a diameter sufficient to pass a guidewire therethrough and/or to introduce one or more fluids through the catheter body 12.

The catheter body 12 may include one or more portions constructed from metals, polymers, or a combination of polymers and metals. Examples of materials that may be used include stainless steel (SST), nickel titanium (Nitinol), or polymers. Examples of other metals which may be used include, super elastic nickel titanium, shape memory nickel titanium, Ti-Ni, nickel titanium, approximately, 55-60 wt. % Ni, Ni--Ti--Hf, Ni--Ti--Pd, Ni--Mn--Ga, Stainless Steel (SST) of SAE grade in the 300 to 400 series e.g., 304, 316, 402, 440, MP35N, and 17-7 precipitation hardened (PH) stainless steel, other spring steel or other high tensile strength material or other biocompatible metal material.

The catheter body 12 may include one or more tube components having one or more cut patterns therein to provide graduated transitions in bending flexibility, as measured by pushability, kink resistance, axial torque transmission for rotational response, and/or torque to failure along a length thereof.

The modulation of flexibility/rigidity across the length of the catheter body 12 can be accomplished in a number of ways. For example, by varying spiral-cut pattern variables (pitch, interruptions) and transitioning between spiral-cut patterns the flexibility/rigidity of a tubular component of catheter body 12 may be controlled. In addition, the spiral-cut pattern allows the cross-sectional diameter of the lumen to be maintained when the tubular module is bent or curved. Spiral-cut sections having different cut patterns may be distributed along the length of the tubular module. The spiral-cut patterns may be continuous or discontinuous along the length of the catheter body 12. For example, there may be 1, 2, 3, 4, 5, 6, 7, ... n spiral-cut sections along the length of the device. The spiral-cut sections may be continuous or interrupted. Within each section a constant cut pattern may be present, but across different sections within a tubular module, the cut patterns may vary, e.g., in terms of pitch.

One or more sections of the catheter body 12 may also contain a variable pitch pattern within the particular section. Each spiral-cut section may have a constant pitch, e.g., in the range of from about 0.05 mm to about 10 mm, e.g., 0.1 mm, 0.2 mm, 0.3 mm, 0.4 mm, 0.5 mm, 0.6 mm, 0.7 mm, 0.8 mm, 0.9 mm, 1.0 mm, 1.5 mm, 2.0 mm, 2.5 mm, 3.0 mm, 3.5 mm, 4.0 mm, etc. The pitch may also vary within each section. The pitches for different spiral-cut sections may be the same or different compared to other sections of the catheter body 12. Alternatively, the catheter body 12 may be formed from tubular modules have a continuously changing spiral-cut pattern along the length of the catheter. The orientation or handedness of spiral-cut sections in the modules may also vary within the spiral-cut sections.

One or more sections of the catheter body 12 can be coated with a lining that protects the cut-pattern components of the catheter body 12 and facilitates transport of additional tools devices such as guidewires and balloons through the tube components of the catheter body 12 to distal locations. Outer and/or inner lining(s) can be made from a polymer, e.g., by enclosing the tube wall with a co-extruded polymeric tubular structure of single of multiple layers and heat shrinking the tubular structure, or coating the tube wall via a dip coating process. The polymer jacket material can be nylon, polyether block amide, PTFE (polytetrafluoroethylene), FEP (fluorinated ethylene propylene), PFA (perfluoroalkoxy alkane), PET (polyethylene terephthalate) or PEEK (polyether ether ketone). Further, the distal tube portion 120 (or the entire length of catheter 100) may be coated with a hydrophilic polymer coating to enhance lubricity and trackability. Hydrophilic polymer coatings can include, but are not limited to, polyelectrolyte and/or a non-ionic hydrophilic polymer, where the polyelectrolyte polymer can include poly(acrylamide-co-acrylic acid) salts, a poly(methacrylamide-co-acrylic acid) salts, a poly(acrylamide-co-methacrylic acid) salts, etc., and the non-ionic hydrophilic polymer may be poly(lactams), for example polyvinylpyrollidone (PVP), polyurethanes, homo- and copolymers of acrylic and methacrylic acid, polyvinyl alcohol, polyvinylethers, snapic anhydride based copolymers, polyesters, hydroxypropylcellulose, heparin, dextran, polypeptides, etc.

A lubricious coating or film may be added over the catheter body 12 to facilitate movement of the device 10 through blood vessels. The lubricious coating can be composed of, for example, silicone or hydrogel polymers or the like, such as polymer networks of a vinyl polymer, polyalkylene glycols, alkoxypolyethylene glycols or an uncrosslinked hydrogel, e.g., Polyethylene oxide (PEO). The coatings and liners disclosed herein can be applied by a dip coating process or by spraying the coating onto the tube outer and inner surfaces.

Additional features of the device 10 and the catheter body 12 are provided in U.S. Patent Application Serial No. 15/726,024 (U.S. Pat. Pub. No. 2018/0093070), entitled 'MODULAR VASCULAR CATHETER,' the entirety of which is incorporated herein by reference.

The device 10 may include a secondary lumen assembly 22 coupled to the distal segment 18 of the catheter body 12. The secondary lumen assembly 12 may generally include a tube or tube segment 24 defining a lumen therein 26 operable to receive a guidewire or other instrument/auxiliary device therethrough. The tube segment 24 may be constructed and/or have certain features and characteristics similar to the catheter body 12. The tube segment 24 may be attached to an outer surface of the catheter body 12 such that the tube segment 24 is not concentric or coaxial with the catheter body 12, and is instead in a side-by-side configuration.

The tube segment 24 may be partially coupled to the catheter body 12 to enable a degree of rotational freedom, movement, bending, and/or displacement from the catheter body 12. For example, the tube segment 24 may be coupled to the catheter body 12 at a proximal portion of the tube segment 24 to form a proximal joint 28, and may be coupled to the catheter body 12 at a distal portion of the tube segment 24 to form a distal joint 30. A substantial remainder of the tube segment 24 between the proximal and distal joints 28,30 may be unsecured and/or unattached to the catheter body.

The proximal and distal joint 28,30 may include one or more of a variety of different attachment modalities. For example, the joints 28,30 may each include a pre-formed, molded cap that receives a portion of the tube segment 24 therein; the joints 28,30 may be formed from a sheath and/or heat shrink tubing encompassing the ends of the tube segment 24; and/or the joints 28,30 may include an adhesive, fuse and/or weld attachment. For example, the proximal and distal joint 28,30 may be formed by melting or fusing an exterior polymer layer of a tubular component constituting the joints 28,30 or tube segment 24 to the catheter body 12, which may be constructed from a metal such as nitinol.

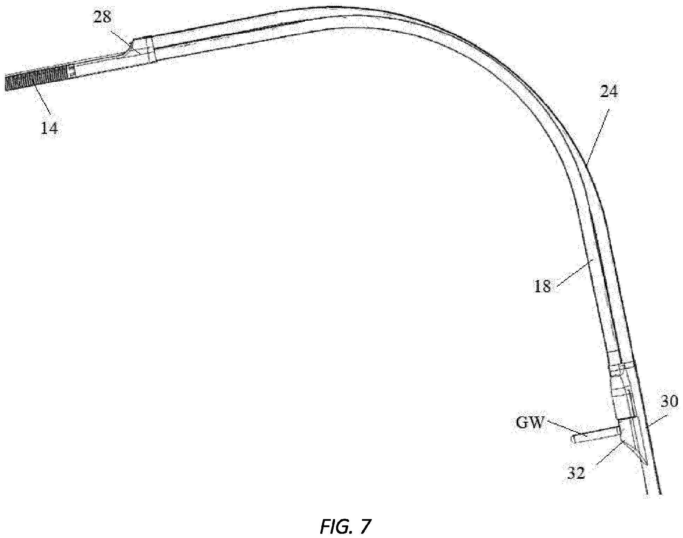

The partial attachment of the secondary lumen assembly 22 to the catheter body 12 provides improved rotational and torqueabiltiy characteristics compared to prior offset dual-lumen catheters that are substantially and/or completely attached along a length thereof. Attempts to rotate such prior devices about the longitudinal axis of the primary lumen/catheter body while in the tortuous vascular anatomy of a patient can result in offset, incongruous, and/or asymmetrical bending at a distal segment thereof. Such incongruous bending and rotation can substantially increase the difficulty for a physician trying to access, treat, or otherwise position a device in a precise location and in a specific rotational orientation. In contrast, the device 10 can be bowed and more readily take on contoured, curved configurations as shown in FIGS. 7-9 for example, due to the freedom of movement between the tube segment 24 and the distal segment 18 of the catheter body 12. The partial attachment between the tube segment 24 and the distal segment 18 of the catheter body 12 allows the two components to shift with respect to one another and follow a path of least resistance when bending around one or more curves in the vasculature.

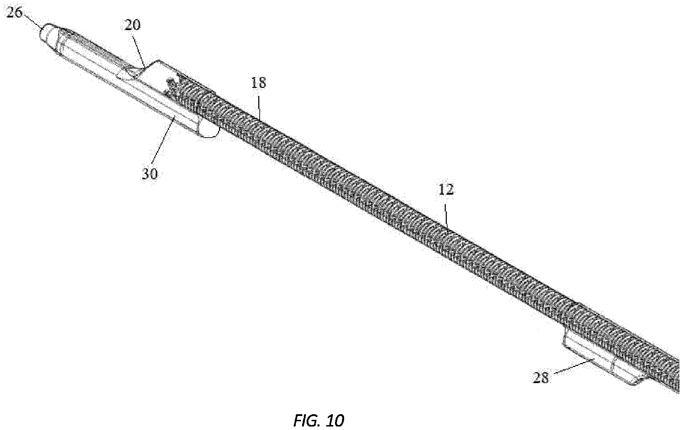

In another example, the device may include proximal and distal joints 28,30 secured and/or attached to the distal segment 18 of the catheter body 12 without the tube segment 24 therebetween, as shown in FIG. 10. In this example, a guidewire (not shown) or other device may be routed through the lumens/openings of the proximal and distal joints 28,30, and exit at the distal end 26. The guidewire would then have the same freedom of movement with respect to the catheter body 12 as described above, allowing independent movement, bending, and rotation thereof during use.

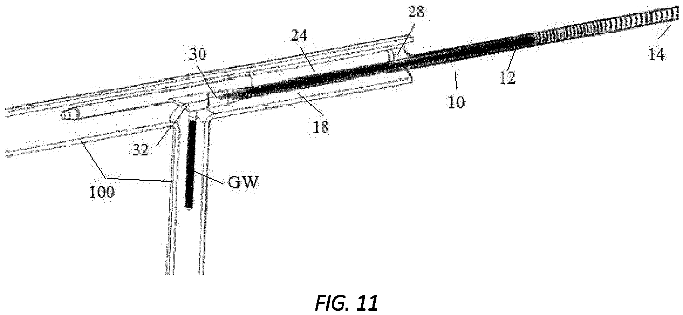

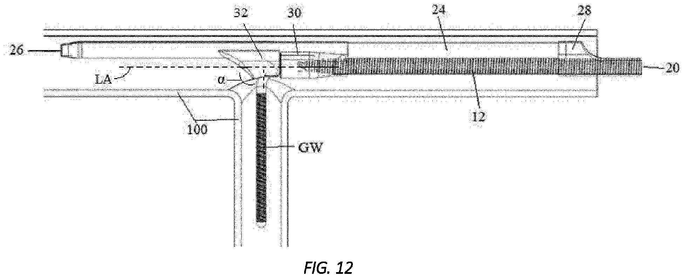

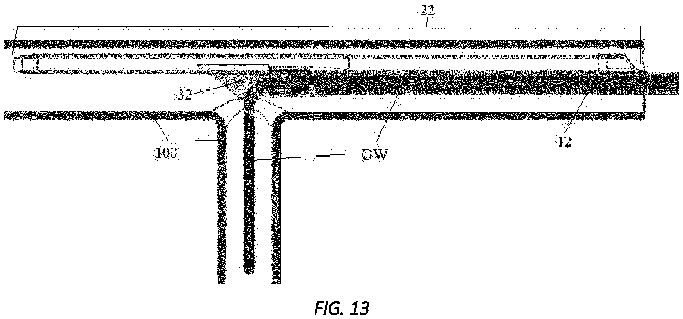

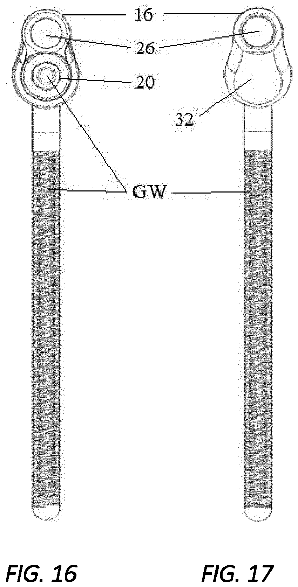

Now referring to FIGS. 11-21, additional examples of the device 10 are shown having a deflection element 32 coupled to the catheter body 12 distal to and or adjacent to the exit of the lumen 20 that can aid in deflecting or otherwise steering a guidewire GW and/or other auxiliary device at a substantial angle away from the longitudinal axis LA of the catheter body 12 and the lumen 20. Such angled exit may be at angle α between 45 degrees and approximately 135 degrees from the longitudinal axis LA of the lumen 20, and can facilitate placement of a guidewire GW or other instrument/auxiliary device into a side branch or other vascular pathway, represented by and generally labeled as '100.'

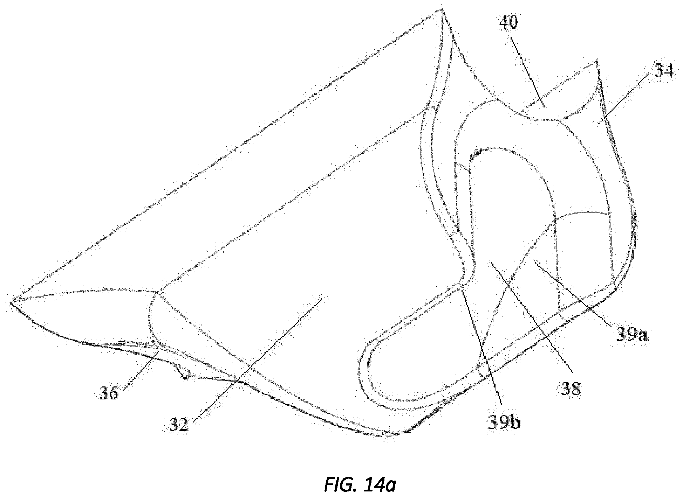

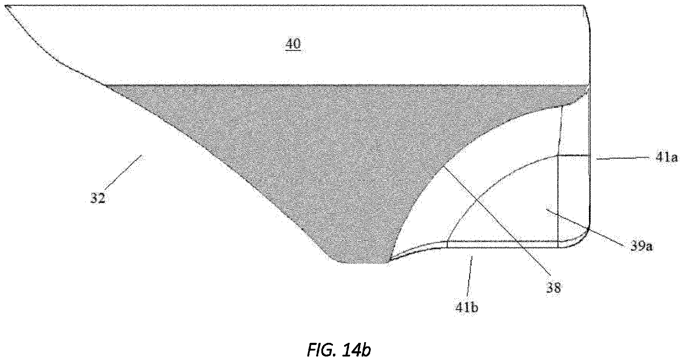

As shown in FIG. 14a-b, the deflection element 32 may include or define a proximal end 34, a distal end 36, and a ramp or contoured surface 38 that deflects a guidewire or auxiliary instrument/device away from the longitudinal axis of the catheter body 12. The contoured surface 38 may include a rounded or arcuate shape to provide the desired angular deflection, and may define an arc between approximately 45 degrees and approximately 135 degrees, as shown in the cross-sectional side view of FIG. 14b.

The deflection element 32 may include or define sidewalls 39a, 39b extending from or bordering the contoured surface 38 to aid in directing or guiding a guidewire or other instrument into a first opening or guidewire entry 41a adjacent to and substantially coaxial with the lumen 20, towards the contoured surface 38, and deflected towards a second opening or guidewire exit 41b substantially perpendicular to the entry 41a to extend outwards from the device 10. In the illustrated example, the first and second openings 41a, 41b form a continuous, open region traversing the deflection element 32, but it is contemplated that additional walls or other structures can form and/or frame the deflected track through which a guidewire or other interventional device could traverse and exit the device 10.

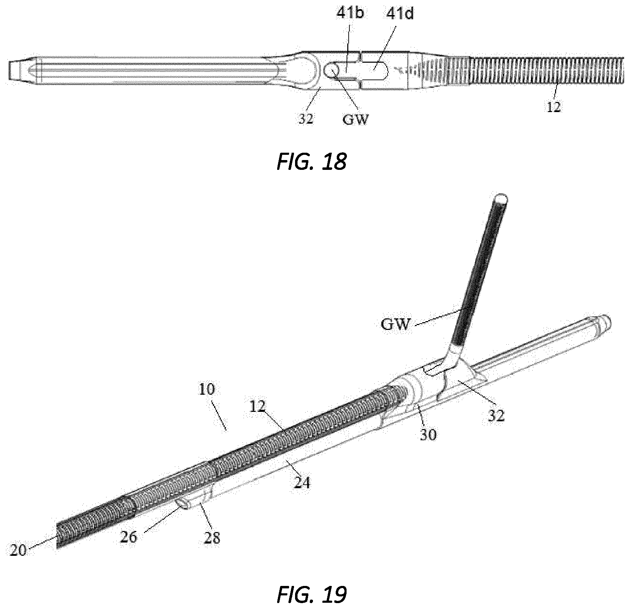

The catheter body 12 may define an opening 41d that is adjacent to and/or coplanar with the guidewire exit opening 41b of the deflection element 32, as shown in FIGS. 18-19. The opening 41d increases the overall space that a guidewire can exit the catheter body 12 at and angle away from the longitudinal axis. Guidewires typically have varying degrees of stiffness along their length, with distal end of guidewires typically being more flexible than proximal portions of the guidewire. The larger exit window formed by openings 41b and 41d allows the stiffer portions of the guidewire to exit the catheter body 12 without requiring a sharper turn or bend that the stiffer guidewire portions could resist. The larger exit thus facilitates easier relative movement between the device 10 and the guidewire when moving or retracting the catheter body 12 from the guidewire.

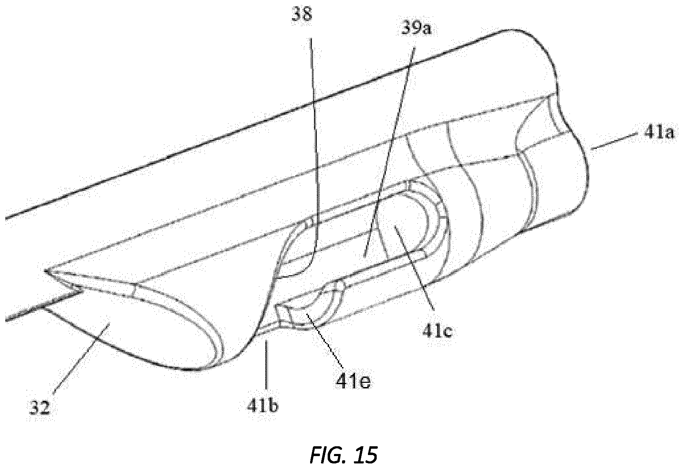

To further aid in routing the stiffer portions of the guidewire through and out of the catheter body 12, the deflection element 32 and or distal exit of the lumen 20 may include one or more flexible or elastic edges that can stretch under the load of the guidewire. In addition, and/or alternatively to the flexible components, the deflection element 32 and or distal exit of the lumen 20 may include one or more rounded interior segments that provide a curved interior surface for the guidewire to traverse when exiting the catheter body rather than just a perpendicular sidewall. For example, the edge or sidewall 41e shown in FIG. 15 having a perpendicular edge that a guidewire would contact when exiting the lumen 20 could include one or more elastic components and/or may be modified in thickness, roundness, or otherwise to reduce friction with the guidewire and/or to prevent the guidewire from kinking.

The deflection element 32 may include or define a complementary surface or segment 40 sized and shaped to matably attach or couple to the secondary lumen assembly 22. In the illustrated example, the surface 40 is cylindrical to accommodate the rounded shape of the tube segment 24.

Continuing to refer to FIG. 15, the deflection element 32 may define a third opening or guidewire exit 41c in a sidewall 39b thereof. The third opening 41c may allow a guidewire to exit the deflection element at an angle substantially perpendicular to the longitudinal axis of the lumen 20 in an alternative plane to that of the first opening 41b. The third opening 41 allows the device 10 to be rotated about a positioned guidewire for retraction of the device 10 form the guidewire while reducing the dislodgment or displacement of the guidewire, as described further herein.

The device 10 may include one or more features to aid a physician in visualizing the location and orientation of the device 10 within the vasculature of a patient, as well as improving the ability of the physician to position guidewires and/or other auxiliary instruments/devices through the lumens and adjacent the desired tissue as desired. For example, the catheter body 12 and/or the deflection element 32 may include one or more radiopaque markers and/or may be constructed from a radiopaque material. The deflection element 32 and/or catheter body 12 may include one or more elastic, deformable, and/or movable components to allow the deflection element 32 to move relative to the catheter body 12 and/or the exit of the lumen 20, thereby providing a physician with a degree of longitudinal maneuverability in placing or directing a guidewire or other or auxiliary instrument/device away from the longitudinal axis of the catheter body 12 and into a side branch or other vascular pathway.

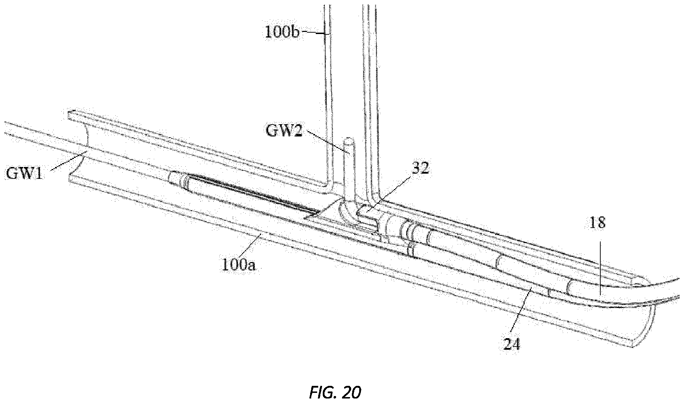

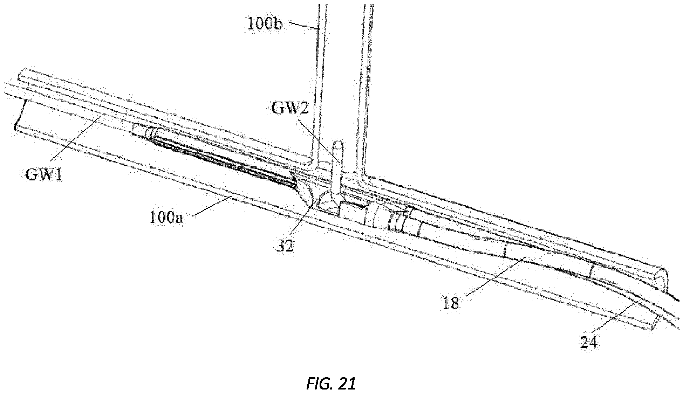

Now referring to FIGS. 20-21, an exemplary use of the device 10 is shown. The device 10 may routed into a primary vessel or vasculature pathway 100a. Insertion and routing of the device 10 into the primary vessel 100a may be facilitated by traversing a first guidewire GW1 routed through the second lumen assembly including tube segment 24 as described herein. The device 10 may be positioned adjacent to or otherwise in proximity to a secondary vessel or side branch vascular pathway 100b. A second guidewire GW2 may be routed through the first lumen 20 of the catheter body 12 towards the deflection element 32. The deflection element 32 may be oriented such that the second opening or guidewire exit 41b is substantially aligned with the secondary vessel 100b. Such positioning and alignment may be achieved through one or more medical imaging modalities. Once the desired positioning has been achieved, the second guidewire GW2 may be routed through the deflection element 32, deflected out of the guidewire exit 41b by the ramp surface 38, and into the secondary vessel 100b. The device 10 may then be rotated about the longitudinal axis of the second guidewire GW2 such that the portion of the second guidewire GW2 extending out of the device 10 is aligned and exiting outward from the third opening 41c in the sidewall 39b of the deflection element, as shown in FIG. 21. The device 10 may then be retracted from the primary vessel 100a while reducing the 'pull' or deflection exerted on the second guidewire GW2 that remains routed in the secondary vessel 100b for subsequent use.

It will be appreciated by persons skilled in the art that the present disclosure is not limited to what has been particularly shown and described herein above. In addition, unless mention was made above to the contrary, it should be noted that all of the accompanying drawings are not to scale. Of note, the system components have been represented where appropriate by conventional symbols in the drawings, showing only those specific details that are pertinent to understanding the embodiments of the present disclosure so as not to obscure the disclosure with details that will be readily apparent to those of ordinary skill in the art having the benefit of the description herein. Moreover, while certain embodiments or figures described herein may illustrate features not expressly indicated on other figures or embodiments, it is understood that the features and components of the examples disclosed herein are not necessarily exclusive of each other and may be included in a variety of different combinations or configurations without departing from the scope and spirit of the disclosure. A variety of modifications and variations are possible in light of the above teachings without departing from the scope and spirit of the disclosure, which is limited only by the following claims.

## Claims

1. A medical device, comprising:
an elongate catheter body defining a proximal segment, a distal segment, and a first lumen therethrough; and
a tube attached to the distal segment of the catheter body, the tube defining a proximal end, a distal end, and a second lumen therethrough;
wherein the proximal end of the tube is attached to the catheter body at a first joint;
wherein the distal end of the tube is attached to the catheter body at a second joint; and
wherein a portion of the tube extending between the first and second joints is movable with respect to the catheter body.

2. The medical device of claim 1, wherein the first joint includes a first cap concentrically mounted onto the proximal end of the tube, and wherein the first cap is attached to the catheter body.

3. The medical device of claim 2, wherein the second joint includes a second cap concentrically mounted onto the distal end of the tube, and wherein the second cap is attached to the catheter body.

4. The medical device of claim 1, wherein at least one of the first and second joints includes attachment of the tube to the catheter body with heat shrink tubing.

5. The medical device of claim 1, wherein the tube is at least partially constructed from a polymer, and wherein at least one of the first and second joints includes attachment of the tube to the catheter body by melt fusing a portion of the tube to a portion of the catheter body.

6. The medical device of claim 1, wherein at least one of the first and second joints includes attachment of the tube to the catheter body with an adhesive.

7. The medical device of claim 1, wherein the first lumen is not concentric with the second lumen.

8. The medical device of claim 1, further comprising a deflection element attached to the distal segment of the catheter body, the deflection element defining an arcuate surface positioned adjacent to the first lumen.

9. The medical device of claim 8, wherein the arcuate surface defines an arc between 45 degrees and 135 degrees.

10. The medical device of claim 8, wherein the deflection element defines a first opening substantially coaxial with the first lumen, a second opening that is substantially perpendicular to the first opening, and wherein the arcuate surface extends between the first and second openings.

11. The medical device of claim 10, wherein the deflection element defines a third opening substantially perpendicular to each of the first and second openings.

12. The medical device of claim 8, wherein the deflection element is radiopaque.

13. The medical device of claim 8, wherein the deflection element is movable with respect to the catheter body.

14. The medical device of claim 8, wherein the deflection element is movable along a longitudinal axis of the catheter body.

15. A medical device, comprising:
an elongate catheter body defining a proximal segment, a distal segment, and a first lumen therethrough; and
a tube attached to the distal segment of the catheter body, the tube defining a second lumen therethrough that is not concentric with the first lumen; and
a deflection element attached to the distal segment of the catheter body, the deflection element defining an arcuate surface positioned adjacent to a distal end of the first lumen, wherein the arcuate surface defines an arc between 45 degrees and approximately 135 degrees.

16. The medical device of claim 15, wherein the deflection element defines a first opening substantially coaxial with the first lumen, a second opening that is substantially perpendicular to the first opening, and wherein the arcuate surface extends between the first and second openings.

17. The medical device of claim 15, wherein the deflection element defines a third opening substantially perpendicular to each of the first and second openings.

18. The medical device of claim 15, wherein the deflection element is radiopaque.

19. The medical device of claim 15, wherein the deflection element is movable with respect to the catheter body.

20. The medical device of claim 15, wherein the deflection element is movable along a longitudinal axis of the catheter body.

21. The medical device of claim 15, wherein at least a portion of the tube is movable independently of the catheter body.
